(19) Europäisches Patentamt
European Patent Office
Office européen des brevets

(11) **EP 4 659 674 A1**

(12) **EUROPEAN PATENT APPLICATION**
published in accordance with Art. 153(4) EPC

(43) Date of publication:
**10.12.2025 Bulletin 2025/50**

(51) International Patent Classification (IPC):
***A61B 6/00*** (2024.01)

(21) Application number: **23919907.8**

(52) Cooperative Patent Classification (CPC):
**A61B 6/4233; A61B 6/00; A61B 6/542**

(22) Date of filing: **29.11.2023**

(86) International application number:
**PCT/JP2023/042780**

(87) International publication number:
**WO 2024/161772 (08.08.2024 Gazette 2024/32)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **02.02.2023 JP 2023014742**

(71) Applicant: **FUJIFILM Corporation
Tokyo 106-8620 (JP)**

(72) Inventor: **TAKAHASHI, Tomoyuki
Ashigarakami-gun, Kanagawa 258-8538 (JP)**

(74) Representative: **Dehns Germany Partnerschaft
mbB
Theresienstraße 6-8
80333 München (DE)**

(54) **RADIOGRAPHY CONTROL DEVICE, METHOD, AND PROGRAM**

(57)    The processor acquires a plurality of radiation images from a plurality of radiation detectors by performing radiography in which a layer structure detector configured by stacking the plurality of radiation detectors is irradiated with radiation emitted from a radiation source and transmitted through an imaging target, determines excess or insufficiency of the radiation at a time of the radiography based on at least one radiation image of the plurality of radiation images, and notifies a determination result of the excess or insufficiency.

**FIG. 3**

RADIOGRAPHY CONTROL DEVICE — 10
- IMAGING CONTROLLER — 21
- DEVICE CONTROLLER — 22
- DETERMINATION UNIT — 23
- DOSE SETTING UNIT — 24
- SUBTRACTION UNIT — 25
- DISPLAY CONTROLLER — 26

EP 4 659 674 A1

**Description**

BACKGROUND OF THE INVENTION

1. Field of the Invention

The present disclosure relates to a radiography control device, a radiography control method, and a radiography control program.

2. Description of the Related Art

In the related art, in radiographic imaging, imaging has been managed using a dose of radiation reaching a radiation detector as an indicator. For example, JP2019-058608A proposes a method of deriving an amount of a signal included in a radiation image based on a distribution of primary radiation of the radiation image based on imaging conditions, or based on the imaging conditions and a body thickness distribution of a subject of the radiation image, outputting a ratio of the derived amount of the signal to an amount of noise included in the radiation image as an index value of a dose of the radiation irradiated during the radiographic imaging, and determining whether the dose of the radiation irradiated during the radiographic imaging is excessive or insufficient based on the index value and a predetermined target value and outputting a determination result.

On the other hand, energy subtraction processing using two radiation images obtained by irradiating a subject with two types of radiation having different energy distributions by using the fact that an attenuation amount of the transmitted radiation differs depending on the substance constituting the subject has been known. As a radiation detector for performing such energy subtraction processing, a layer structure detector has been proposed. The layer structure detector is configured by, for example, stacking two radiation detectors in a layer shape. It is possible to perform energy subtraction imaging with one irradiation of radiation by using such a layer structure detector. In addition, it is also possible to perform simple imaging similar to a case in which only one radiation detector is used, by acquiring the radiation image using only a radiation detector on the side close to the radiation source.

SUMMARY OF THE INVENTION

However, in a case in which the layer structure detector is used, the radiation dose irradiated onto the radiation detector on the side far from the radiation source is smaller than the radiation dose applied to the radiation detector on the side close to the radiation source. Therefore, in a case in which the dose is not appropriately set in a case in which the energy subtraction processing is performed, the radiation dose with which the radiation detector on the side far from the radiation source is irradiated is too small, so that the signal-to-noise ratio (S/N) is decreased. As a result, the image quality of the radiation image acquired by the energy subtraction processing degrades. On the other hand, in a case in which the radiation detector on the side far from the radiation source is irradiated with a sufficient amount of radiation, the pixel value of the radiation image acquired by the radiation detector on the side close to the radiation source may be saturated. Even in this case, the image quality of the radiation image acquired by the energy subtraction processing degrades.

The present disclosure has been made in view of the above circumstances, and an object of the present disclosure is to enable acquisition of a high-quality radiation image in a case in which radiography is performed using a layer structure detector.

According to the present disclosure, there is provided a radiography control device comprising at least one processor, in which the processor is configured to: acquire a plurality of radiation images from a plurality of radiation detectors by performing radiography in which a layer structure detector configured by stacking the plurality of radiation detectors is irradiated with radiation emitted from a radiation source and transmitted through an imaging target; determine excess or insufficiency of the radiation at a time of the radiography by using at least one radiation image of the plurality of radiation images as a radiation image for determination; and notify of a determination result of the excess or insufficiency.

In the radiography control device according to the present disclosure, the processor may be configured to determine the excess or insufficiency of the radiation by using a radiation image acquired by one radiation detector other than a radiation detector closest to the radiation source among the plurality of radiation detectors as the radiation image for determination.

In addition, in the radiography control device according to the present disclosure, the processor may be configured to: specify a subject region of the radiation image for determination; and determine that a dose of the radiation with which the one radiation detector is irradiated is insufficient in a case in which a pixel value of at least a part of the subject region is less than a reference pixel value or a granular noise amount of at least a part of the subject region exceeds a reference amount.

In addition, in the radiography control device according to the present disclosure, the processor may be configured to: detect an artificial object region included in the radiation image for determination; and specify a region excluding the artificial object region as the subject region.

In addition, in the radiography control device according to the present disclosure, the processor may be configured to: derive a minified image of the radiation image for determination; and determine whether or not the pixel value of at least a part of the subject region is less than the reference pixel value based on the minified image.

In addition, in the radiography control device according to the present disclosure, the processor may be configured to determine whether or not the granular noise amount of at least a part of the subject region exceeds the reference amount, based on a high-frequency component of the radiation image for determination.

In addition, in the radiography control device according to the present disclosure, the processor may be configured to, in a case in which it is determined that the dose of the radiation with which the one radiation detector is irradiated is not insufficient, determine the excess or insufficiency of the radiation by using a radiation image acquired by the radiation detector closest to the radiation source among the plurality of radiation detectors as an additional radiation image for determination.

In addition, in the radiography control device according to the present disclosure, the processor may be configured to determine that the dose is excessive in a case in which a pixel value of at least a part of a region corresponding to the subject region in the additional radiation image for determination is saturated.

In addition, in the radiography control device according to the present disclosure, the processor may be configured to: derive a dose setting value for setting the pixel value of the subject region to a target value in a case in which it is determined that the dose of the radiation is excessive or insufficient; and notify of the dose setting value.

According to the present disclosure, there is provided a radiography control method comprising: acquiring a plurality of radiation images from a plurality of radiation detectors by performing radiography in which a layer structure detector configured by stacking the plurality of radiation detectors is irradiated with radiation emitted from a radiation source and transmitted through an imaging target; determining excess or insufficiency of the radiation at a time of the radiography by using at least one radiation image of the plurality of radiation images as a radiation image for determination; and notifying of a determination result of the excess or insufficiency.

According to the present disclosure, there is provided a radiography control program causing a computer to execute: a procedure of acquiring a plurality of radiation images from a plurality of radiation detectors by performing radiography in which a layer structure detector configured by stacking the plurality of radiation detectors is irradiated with radiation emitted from a radiation source and transmitted through an imaging target; a procedure of determining excess or insufficiency of the radiation at a time of the radiography by using at least one radiation image of the plurality of radiation images as a radiation image for determination; and a procedure of notifying of a determination result of the excess or insufficiency.

According to the present disclosure, it is possible to acquire a high-quality radiation image in a case in which radiography is performed using a layer structure detector.

BRIEF DESCRIPTION OF THE DRAWINGS

Fig. 1 is a schematic block diagram showing a configuration of a radiography system to which a radiography control device according to an embodiment of the present disclosure is applied.

Fig. 2 is a diagram showing a schematic configuration of a radiography control device according to the present embodiment.

Fig. 3 is a diagram showing a functional configuration of the radiography control device according to the present embodiment.

Fig. 4 is a diagram showing a histogram for describing specifying of a subject region.

Fig. 5 is a diagram for describing specifying of a subject region.

Fig. 6 is a diagram for describing acquisition of a profile of a pixel value.

Fig. 7 is a diagram showing a profile of a pixel value.

Fig. 8 is a diagram for describing derivation of a dose setting value in a case of an insufficient dose.

Fig. 9 is a diagram for describing derivation of a dose setting value in a case of an excessive dose.

Fig. 10 is a diagram showing a display screen in a case in which a dose is appropriate.

Fig. 11 is a diagram showing a display screen in a case of an insufficient dose.

Fig. 12 is a diagram showing a display screen in a case of an excessive dose.

Fig. 13 is a flowchart showing processing performed in the present embodiment.

DESCRIPTION OF THE PREFERRED EMBODIMENTS

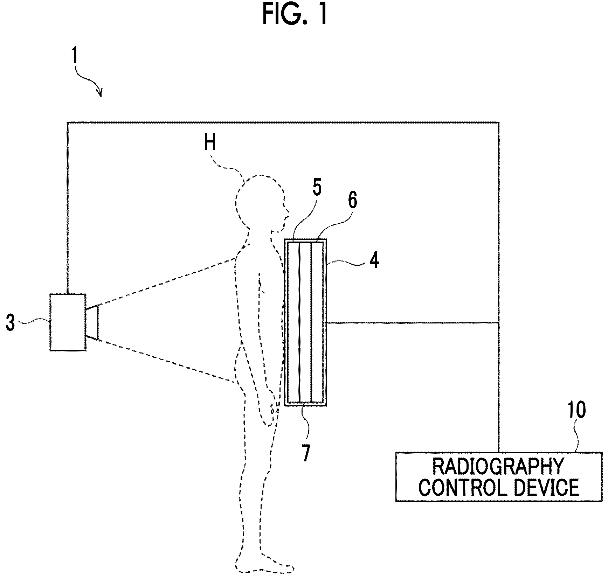

In the following, an embodiment of the present disclosure will be explained with reference to the drawings. Fig. 1 is a schematic block diagram showing a configuration of a radiography system to which a radiography control device according to the present embodiment of the present disclosure is applied. As shown in Fig. 1, the radiography system according to the present embodiment comprises an imaging apparatus 1 and a radiography control device 10 according to the present embodiment.

The imaging apparatus 1 comprises a radiation source 3 and a layer structure detector 4. The layer structure detector 4 is configured by stacking a first radiation detector 5, a radiation energy conversion filter 7 consisting of a copper plate or the like, and a second radiation detector 6 in order from a side close to the radiation source 3.

By using such a layer structure detector 4, in the imaging apparatus 1, it is possible to perform energy subtraction by a so-called one-shot method of irradiating the first radiation detector 5 and the second radiation detector 6 with radiation, such as X-rays, which is emitted from the radiation source 3 and transmitted through a subject H, which is an imaging target, with different energies. In addition, in a case in which only the first radiation detector 5 of the layer structure detector 4 is used, it is possible to acquire a radiation image by performing simple imaging of the subject H.

Here, the energy subtraction is processing of generating an image in which different tissues (for example, a soft part and a bone part) in the subject are extracted by using two radiation images obtained by irradiating the subject with two types of radiation having different energy distributions by using the fact that an attenuation amount of the transmitted radiation differs depending on the substance constituting the subject.

The first and second radiation detectors 5 and 6 can perform recording and reading-out of the radiation image repeatedly. A so-called direct-type radiation detector that directly receives irradiation of the radiation and generates an electric charge may be used, or a so-called indirect-type radiation detector that converts the radiation into visible light and then converts the visible light into an electric charge signal may be used. In addition, as a method for reading out a radiation image signal, it is desirable to use a so-called thin film transistor (TFT) readout method in which the radiation image signal is read out by turning a TFT switch on and off, or a so-called optical readout method in which the radiation image signal is read out by irradiation of read out light. However, other methods may also be used without being limited to these methods.

In the radiography control device 10 according to the present embodiment, the first and second radiation detectors 5 and 6 included in the above-described layer structure detector 4 are switched for use according to the imaging purpose. That is, in a case in which the imaging purpose is simple imaging for acquiring one radiation image of the subject H, only the first radiation detector 5 on a side close to the radiation source 3 is used. On the other hand, in a case in which the imaging purpose is energy subtraction imaging, both the first radiation detector 5 and the second radiation detector 6 are used.

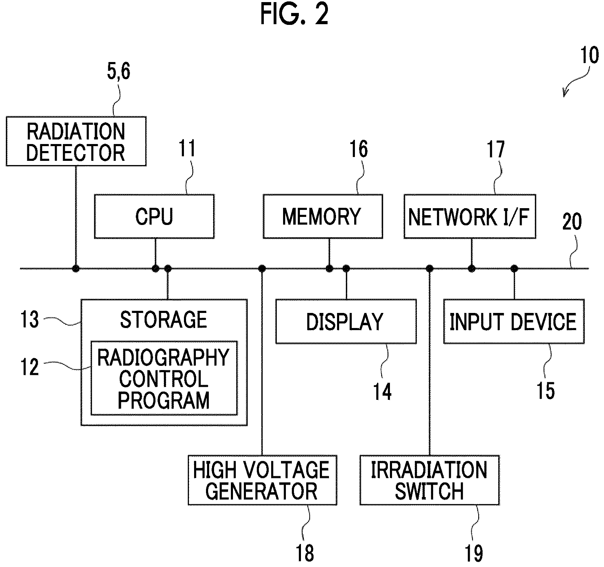

Next, the radiography control device according to the present embodiment will be described. First, a hardware configuration of the radiography control device according to the present embodiment will be described with reference to Fig. 2. As shown in Fig. 2, the radiography control device 10 is a computer, such as a workstation, a server computer, and a personal computer, and comprises a central processing unit (CPU) 11, a non-volatile storage 13, and a memory 16 as a temporary storage area. In addition, the radiography control device 10 comprises a display 14, such as a liquid crystal display, an input device 15, such as a keyboard and a mouse, and a network interface (I/F) 17 connected to a network (not shown). In addition, the radiography control device 10 comprises a high voltage generator 18 and an irradiation switch 19 connected to the radiation source 3. The CPU 11, the storage 13, the display 14, the input device 15, the memory 16, the network I/F 17, the high voltage generator 18, and the irradiation switch 19 are connected to a bus 20. In addition, the radiation detectors 5 and 6 are also connected to the bus 20. It should be noted that the CPU 11 is an example of a processor according to the present disclosure.

The storage 13 is realized by a hard disk drive (HDD), a solid state drive (SSD), a flash memory, and the like. A radiography control program 12 installed in the radiography control device 10 is stored in the storage 13 as a storage medium. The CPU 11 reads out the radiography control program 12 from the storage 13, loads the radiography control program 12 in the memory 16, and executes the loaded radiography control program 12.

The high voltage generator 18 boosts an input voltage using a transformer to generate a high tube voltage, and supplies the high tube voltage to the radiation source 3 through a high voltage cable.

The irradiation switch 19 is, for example, a two-stage push switch operated by an operator such as a radiologist, and pressing it to the first stage generates a warm-up start signal for starting warm-up of the radiation source 3 and pressing it to second stage generates an irradiation start signal for starting irradiation of the radiation source 3.

The radiography control program 12 is stored in a storage device of the server computer connected to the network or in a network storage in a state of being accessible from the outside, and is downloaded and installed in the computer that configures the radiography control device 10 in response to the request. Alternatively, the radiography control program 12 is distributed in a state of being recorded on a recording medium, such as a digital versatile disc (DVD) or a compact disc read only memory (CD-ROM), and is installed in the computer that configures the radiography control device 10 from the recording medium.

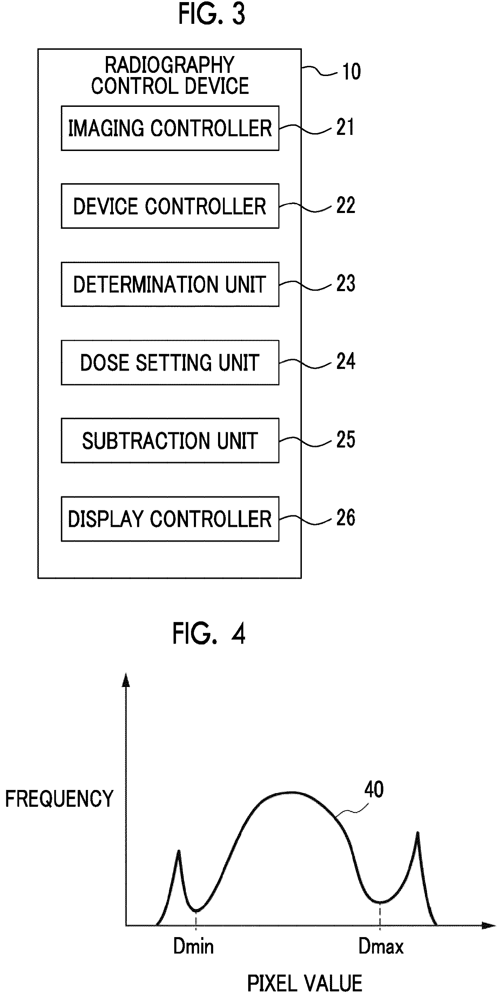

Next, a functional configuration of the radiography control device according to the present embodiment will be described. Fig. 3 is a diagram showing the functional configuration of the radiography control device according to the present embodiment. As shown in Fig. 3, the radiography control device 10 comprises an imaging controller 21, a device controller 22, a determination unit 23, a dose setting unit 24, a subtraction unit 25, and a display controller 26. Then, the CPU 11 executes the radiography control program 12 to function as the imaging controller 21, the device controller 22, the determination unit 23, the dose setting unit 24, the subtraction unit 25, and the display controller 26.

The imaging controller 21 controls radiography by irradiating the subject H with radiation. Specifically, the radiation source 3 is driven by controlling a tube voltage that determines an energy spectrum of radiation emitted from the radiation source 3, a tube current that determines an irradiation amount per unit time, the start, stop, or end of irradiation of the radiation source 3, and an irradiation time of the radiation. That is, the imaging controller 21 starts the supply of power from the high voltage generator 18 to the radiation source 3 in a case in which the irradiation start signal is received from the irradiation switch 19, stops the supply of power from the high voltage generator 18 to the radiation source 3 in a case in which the emitted dose reaches the target dose based on the imaging condition, and stops the irradiation of the radiation by the radiation source 3.

The storage 13 stores several types of imaging conditions, such as a tube voltage and a tube current, in advance according to the imaging part or the like. The imaging conditions are manually set by the operator through the input device 15. The imaging controller 21 emits radiation based on the tube voltage and the tube current irradiation time product of the set imaging conditions.

On the other hand, the imaging controller 21 acquires the imaging purpose. Examples of the imaging purpose include simple imaging of acquiring one radiation image of the subject H and energy subtraction imaging. The imaging purpose is input by the operator through the input device 15. In a case in which the imaging purpose is simple imaging, only the first radiation detector 5 on the side close to the radiation source 3 in the layer structure detector 4 is used to acquire the radiation image (hereinafter, referred to as a simple radiation image G0). On the other hand, in a case in which the imaging purpose is energy subtraction imaging, both the first radiation detector 5 and the second radiation detector 6 are used, and the first radiation detector 5 acquires a first radiation image G1 and the second radiation detector 6 acquires a second radiation image G2.

The device controller 22 controls operations of the first and second radiation detectors 5 and 6 in response to an input operation from the operator via the input device 15. Specifically, the device controller 22 performs various controls such as turning on and off the power of the radiation detectors 5 and 6 and mode switching to a standby mode or an imaging mode. In addition, the device controller 22 preferably has a function of performing various types of image processing such as offset correction, sensitivity correction, and defect correction on the radiation images acquired by the first and second radiation detectors 5 and 6.

The determination unit 23 uses at least one radiation image of the first radiation image G1 acquired by the first radiation detector 5 or the second radiation image G2 acquired by the second radiation detector as a radiation image for determination, determines whether the dose of the radiation in the radiography is excessive or insufficient, and derives a determination result of the excess or insufficiency.

Here, in a case in which the simple imaging is performed, the determination unit 23 determines the excess or insufficiency of the dose by using the simple radiation image G0 acquired by the first radiation detector 5 as the radiation image for determination. On the other hand, in a case in which the energy subtraction imaging is performed, the determination unit 23 determines the excess or insufficiency of the dose by using the first radiation image G1 acquired by the first radiation detector 5 and the second radiation image G2 acquired by the second radiation detector 5 as the radiation images for determination, respectively.

In a case in which the simple imaging is performed, the excess or insufficiency of the dose may be determined using the method described in JP2019-058608A. That is, the determination unit 23 may derive the amount of signal included in the simple radiation image G0 based on the distribution of the primary rays of the simple radiation image G0 based on imaging conditions, or based on the imaging conditions and the body thickness distribution of the subject H of the simple radiation image G0, output a ratio of the derived amount of signal to the amount of noise included in the simple radiation image G0 as an index value of the dose of the radiation irradiated during the simple imaging, and determine the excess or insufficiency of the dose of the radiation based on the index value and the target dose based on the imaging conditions.

In the following, the determination of the excess or insufficiency of the dose in a case in which the energy subtraction imaging is performed will be described. The determination unit 23 specifies a subject region of the second radiation image G2 acquired by the second radiation detector 6 on the side away from the radiation source 3 in the radiation image for determination. The determination unit 23 specifies the subject region of the second radiation image G2 based on a histogram of the second radiation image G2. Fig. 4 is a diagram showing a histogram for describing specifying of a subject region. As shown in Fig. 4, in a histogram 40, a lateral axis represents a signal value of the radiation image, and a vertical axis represents a frequency of the signal value. Here, in the radiation image, a region (direct radiation region) where radiation does not pass through the subject H but is directly irradiated onto the radiation detector has a high density in the radiation image. In addition, in a case in which imaging is performed using an irradiation field stop, the pixel value of the radiation image is 0 because the region other than the irradiation field is not irradiated with the radiation in the radiation detector. In addition, in a case in which an artificial object made of metal or the like, such as a screw, is included in the subject H, the artificial object is less likely to transmit the radiation, and thus has high brightness in the radiation image.

Therefore, the determination unit 23 sets, for example, a minimum density value Dmin of 10% to 20% from a low density (high brightness) side and sets a maximum density value Dmax of 10% to 20% from a high density (low brightness) side in the histogram 40. Then, the determination unit 23 specifies, in the second radiation image G2, a region having a density in a range from the minimum density value Dmin to the maximum density value Dmax as the subject region.

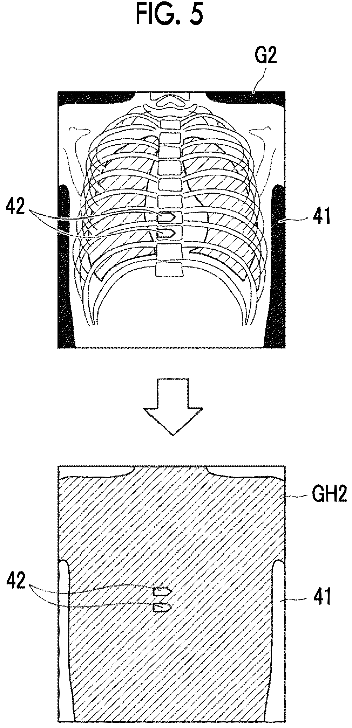

Fig. 5 is a diagram for describing specifying of the subject region. As shown in Fig. 5, a region excluding a direct radiation region 41 and a region 42 of an artificial object, such as a screw embedded in the vertebra, in the second radiation image G2 is specified as a subject region GH2. In a case in which the second radiation image G2 shown in Fig. 5 is acquired, the irradiation field stop is not used.

For example, as described in JP2018-033745A, the subject region may be specified by discriminating a direct radiation region and an irradiation field region in the radiation image using a machine learning discriminator. In this case, the discriminator is generated by machine learning using a large number of radiation images including the irradiation field region and the direct radiation region. In addition, an artificial object region in the radiation image may be discriminated using the discriminator that has been subjected to machine learning to discriminate the artificial object region, and the subject region may be specified by excluding the artificial object region from the radiation image.

Here, since the second radiation detector 6 is located on a side far from the radiation source 3 in the layer structure detector 4, the amount of radiation irradiated onto the second radiation detector 6 is smaller than that of the first radiation detector 5. Therefore, in a case in which the imaging part is the chest, the dose may be insufficient in the mediastinum and the subdiaphragmatic region even though the dose in the lung field is sufficient. In a case in which the dose is insufficient, the pixel value is smaller in the region where the dose is insufficient than in the region where the dose is appropriate. In addition, in a case in which the dose is insufficient, the granular noise is larger in a region where the dose is insufficient than in a region where the dose is appropriate. Therefore, the determination unit 23 determines whether or not a pixel value of at least a part of the subject region GH2 of the second radiation image G2 is less than a reference pixel value or a granular noise amount of the subject region GH2 exceeds a reference amount.

In order to determine whether or not the pixel value of at least a part of the subject region GH2 is less than the reference pixel value, it is preferable to minify the second radiation image G2 to, for example, about 1/4 to 1/16 in both the vertical and horizontal directions to reduce the influence of the granular noise of the radiation included in the subject region GH2, and to determine whether or not the pixel value of at least a part of the subject region GH2 in the minified second radiation image G2 is less than the reference pixel value. In the present embodiment, for example, the determination unit 23 calculates the minimum value of the pixel value of the minified subject region GH2, and in a case in which the minimum value of the pixel value is less than the reference value, determines that the dose is insufficient. The region for determining insufficient dose may be only the region of the mediastinum and the subdiaphragmatic region in which the dose is small in the subject region GH2.

In addition, in order to determine whether or not the granular noise of the subject region GH2 exceeds the reference value, it is preferable to derive a high-frequency component of the subject region GH2, derive a standard deviation of a signal value of the high-frequency component, and determine whether or not the standard deviation is equal to or greater than a predetermined threshold value. For example, in a case in which the standard deviation is equal to or greater than the threshold value, the granular noise exceeds the reference value, and thus, it is sufficient to determine that the dose is insufficient. The high-frequency component is, for example, a frequency component of 1/2 or more of the Nyquist frequency of the original image (that is, the second radiation image G2), but the present disclosure is not limited to this. In addition, the high-frequency component can be derived, for example, by minifying the second radiation image G2 to 1/2, enlarging the minified image to the original size, and subtracting the enlarged image from the second radiation image G2.

In a case in which it is determined that the dose is insufficient, the determination unit 23 outputs a determination result of the dose insufficiency to the dose setting unit 24 and the display controller 26.

On the other hand, since the first radiation detector 5 on the side close to the radiation source 3 is irradiated with a larger dose of the radiation than the second radiation detector 6, even in a case in which the second radiation image G2 is not insufficient in dose, the pixel value on the high density side is saturated in the first radiation image G1 acquired by the first radiation detector 5. Therefore, in a case in which it is determined that the dose is not insufficient, the determination unit 23 specifies a region GH1 (not shown) corresponding to the subject region GH2 in the first radiation image G1. Then, the determination unit 23 determines whether or not the pixel value of at least a part of the region GH1 is saturated. In the present embodiment, for example, in a case in which a region in which the pixel value is saturated is present in the region GH1 of the first radiation image G1, the determination unit 23 determines that the dose is excessive, and outputs a determination result to the dose setting unit 24 and the display controller 26. The region for determining the excess dose may be only the lung field in the region GH1. Here, the pixel value being saturated refers to a state in which the pixel value does not increase any further even in a case in which the dose of the radiation is increased. The pixel value (saturated pixel value) of the saturated pixel may be smaller than the maximum value that a radiation image can take.

In a case in which the dose is neither insufficient nor excessive, the determination unit 23 may output information representing that the dose is appropriate to the display controller 26.

In a case in which the determination unit 23 determines that the dose of the radiation is excessive or insufficient, the dose setting unit 24 derives a dose setting value for setting the pixel value of the subject region to a target value.

Here, in a case in which a pixel value B is obtained in a certain pixel in a case of imaging with a dose value X (mAs), the setting of the dose value in a case in which the pixel value B is increased by $\Delta B$ to set the pixel value to B + $\Delta B$ will be described. For example, in a case in which the dose reaching the radiation detector is in a four-digit range of 0.003 mR to 30 mR and the logarithm of the reaching dose is assigned to 14 bits (pixel value 0 to 16383), the pixel value can be obtained by the following Equation (1).

$$\text{Pixel value} = (\log 10((\text{reaching dose mR})/0.3)+2)/4*16383 \quad (1)$$

In a case of applying to Equation (1), in a case in which the reaching dose is 3 mR, the pixel value is 12287(= (log10(3/0.3)+2)/4*16383).

Here, a case in which the pixel value of 12287 is increased by 1000 to 13287 is considered. In a case in which the above Equation (1) is solved for the reaching dose with the pixel value of 13287, the reaching dose mR $\approx$ 5.26 is obtained. That is, in a case in which the reaching dose is changed from 3 mR to 5.26 mR, a desired pixel value is obtained. Therefore, the dose setting value is derived by multiplying the dose value X (mAs) set at the time of imaging by 5.26/3 (X*5.26/3(mAs)), and the imaging is performed with the derived dose setting value, so that the pixel value of the subject region can be set to the target value.

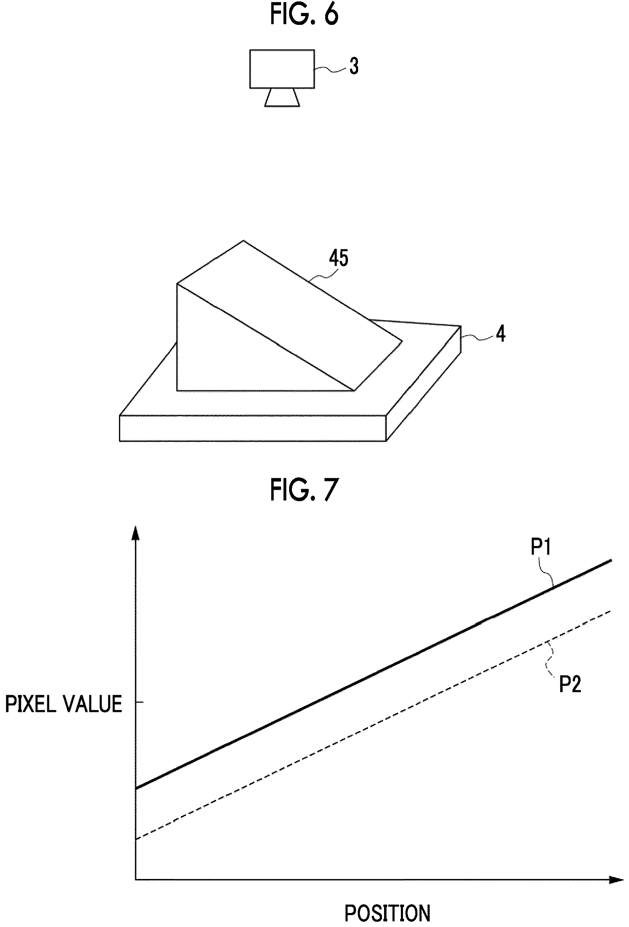

In the present embodiment, in order to derive the dose setting value, as shown in Fig. 6, in a case in which the radiography is performed by placing a thing 45 of which the thickness gradually changes on the layer structure detector 4, profiles of the pixel values obtained in the first radiation detector 5 and the second radiation detector 6 in an inclined direction of the thing 45 are acquired. Fig. 7 is a diagram showing profiles P1 and P2 of pixel values obtained in the first radiation detector 5 and the second radiation detector 6.

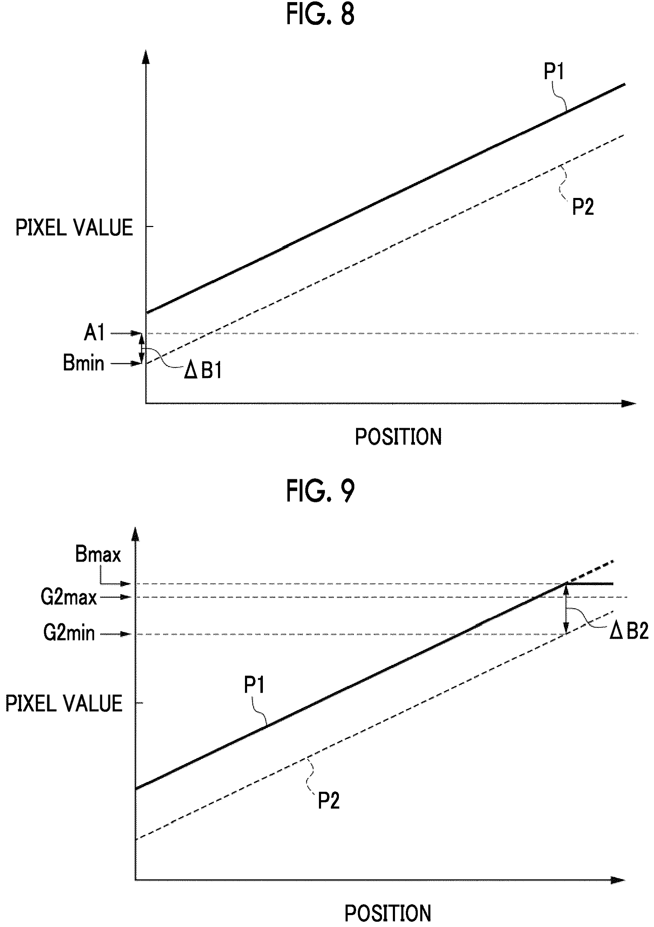

First, a case in which the dose of the radiation with which the second radiation detector 6 is irradiated is insufficient will be described. First, as shown in Fig. 8, a reference value A1 of the pixel value is set in the profile P2 of the pixel value obtained in the second radiation detector 6. In a case in which the minimum value of the pixel value is denoted by Bmin, the dose setting unit 24 derives the dose setting value such that the pixel value Bmin is the reference value A1.

That is, in a case in which a difference between the minimum value Bmin of the pixel value and the reference value A1 is $\Delta$B1, a reaching dose R1 for the reference value A1 is derived by solving the reaching dose in Equation (1) in which the pixel value is set to the reference value A1. In addition, in Equation (1), a reaching dose R2 for the minimum value Bmin is derived by solving the reaching dose with the pixel value as the minimum value Bmin. Then, the dose setting value is derived by multiplying the dose value X set at the time of imaging by (R1/R2).

Next, a case in which the dose of the radiation with which the first radiation detector 5 is irradiated is excessive will be described. As shown in Fig. 9, a saturated pixel value which is a pixel value of a saturated pixel of the first radiation image G1 is set to Bmax, a minimum pixel value of a region of the second radiation image G2 corresponding to a region consisting of the saturated pixels in the first radiation image G1 is set to G2min, and a maximum pixel value of the second radiation image G2 is set to G2max in the first profile P1 and the second profile P2 as shown in Fig. 9. Then, the difference $\Delta$B2 = Bmax - G2min is set. In this case, a pixel value assumed in a case in which the pixel corresponding to the pixel value G2max is not saturated in the first radiation image G1 is G2max + $\Delta$B2. In a case in which G2max + $\Delta$B2 does not exceed the saturated pixel value Bmax, the region GH1 corresponding to the subject region GH2 in the first radiation detector 5 is not saturated.

Therefore, the dose setting unit 24 derives a dose setting value such that G2max + $\Delta$B2 becomes Bmax. That is, a reaching dose R3 for G2max + $\Delta$B2 is derived by solving the reaching dose for G2max + $\Delta$B2 by setting the pixel value to G2max + ΔB2 in Equation (1). In addition, in a case in which the pixel value is set as Bmax in Equation (1) and the reaching dose is solved, a reaching dose R4 for Bmax is derived by solving the reaching dose by setting the pixel value to Bmax in Equation (1). Then, the dose setting value is derived by multiplying the dose value X set at the time of imaging by (R4/R3).

By performing the radiography according to the dose setting value set in this manner, in the first radiation image G1 and the second radiation image G2 acquired by the first radiation detector 5 and the second radiation detector 6, there is no excess or insufficiency of the dose with respect to the subject region. Therefore, the pixel value of the subject region can be set as the target value, and as a result, the first and second radiation images G1 and G2 can be acquired without an increase in noise and saturation of the pixel value due to the insufficient dose.

The dose setting unit 24 outputs the set dose setting value to the display controller 26.

In a case in which the imaging purpose is energy subtraction imaging, the subtraction unit 25 derives a bone part image Gb in which only the bone part of the subject H included in the first radiation image G1 and the second radiation image G2 is extracted and a soft part image Gs in which only the soft part is extracted by performing weighting subtraction between corresponding pixels of the first radiation image G1 acquired by the first radiation detector 5 and the second radiation image G2 acquired by the second radiation detector 6, as shown in the following Equations (2) and (3). Note that $\alpha1$ and $\alpha2$ in Equation (2) and Equation (3) are weighting coefficients and are derived based on an attenuation coefficient according to the radiation energy of the soft part and the bone part of the subject H.

$$Gb(x,y) = G1(x,y) - \alpha1 \times G2(x,y) \quad (2)$$

$$Gs(x,y) = G2(x,y) - \alpha2 \times G1(x,y) \quad (3)$$

The display controller 26 displays the acquired radiation image on the display 14. That is, in a case in which the imaging purpose is simple imaging, the display controller 26 displays the simple radiation image G0 acquired by the first radiation detector 5 on the display 14. In a case in which the imaging purpose is energy subtraction imaging, the display controller 26 displays the bone part image Gb and the soft part image Gs on the display 14. In addition, in the present embodiment, the display controller 26 gives a notification of the determination result of the excess or insufficiency of the dose by displaying the determination result on the display 14.

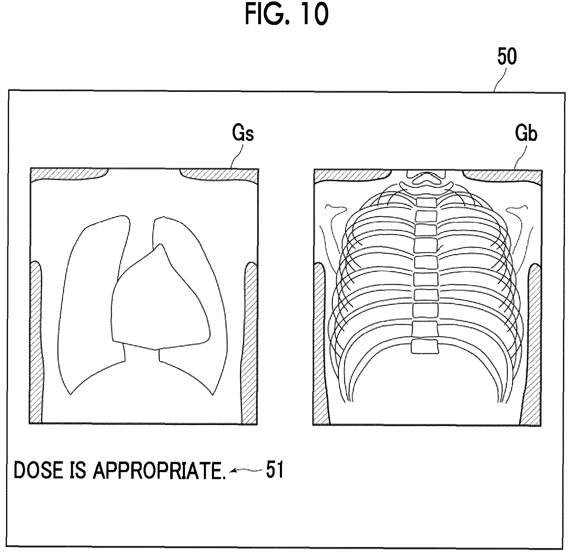

Fig. 10 is a diagram showing a display screen in a case in which a dose is appropriate. As shown in Fig. 10, the display screen 50 displays the bone part image Gb, the soft part image Gs, and information 51 representing that the dose at the time of imaging is appropriate.

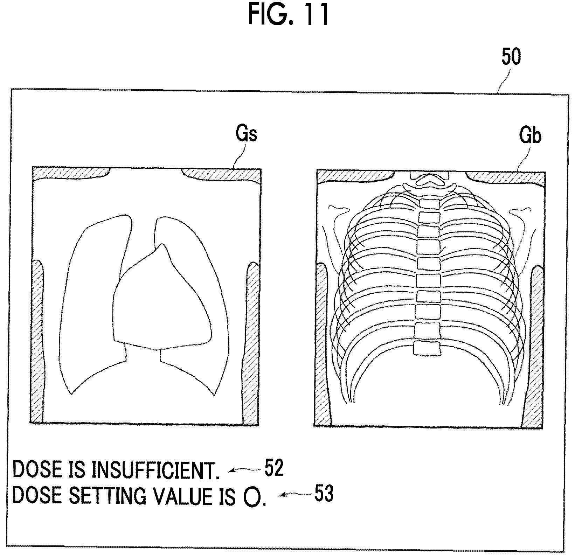

Fig. 11 is a diagram showing a display screen in a case of an insufficient dose. As shown in Fig. 11, the display screen 50 displays the bone part image Gb, the soft part image Gs, information 52 representing that the dose is insufficient at the time of imaging, and information 53 representing the dose setting value at the time of next imaging derived by the dose setting unit 24.

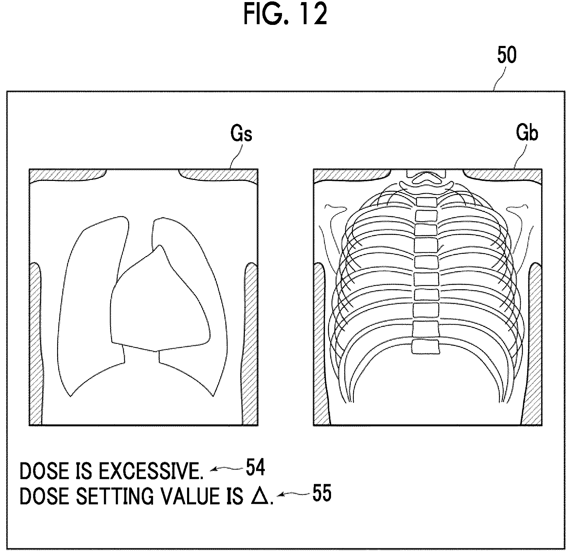

Fig. 12 is a diagram showing a display screen in a case of an excessive dose. As shown in Fig. 12, the display screen 50 displays the bone part image Gb, the soft part image Gs, information 54 representing that the dose is excessive at the time of imaging, and information 55 representing the dose setting value at the time of next imaging derived by the dose setting unit 24.

In a case in which the simple imaging is performed, the display screen 50 displays the simple radiation image G0 acquired by the simple imaging and information representing the excess or insufficiency of the dose are displayed.

In addition, in the above description, the notification is made by displaying the excess or insufficiency of the dose on the display screen 50, but the present disclosure is not limited thereto. The notification may be made by voice or may be made by both voice and display.

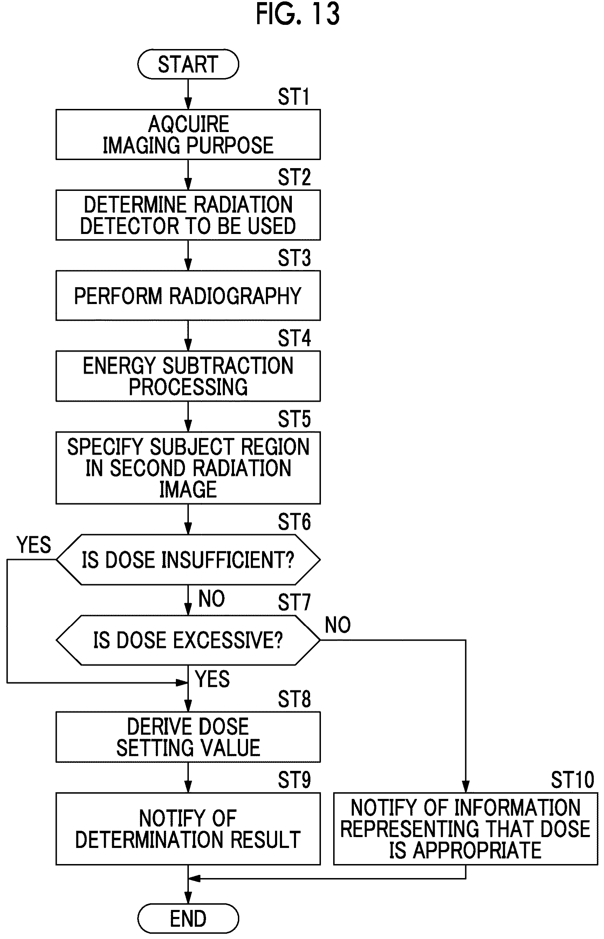

Next, processing performed in the present embodiment will be described. Fig. 13 is a flowchart showing the processing performed in the present embodiment. Here, processing in a case in which the imaging purpose is energy subtraction imaging will be described, and processing in a case in which the imaging purpose is simple imaging is the same as the processing described in JP2019-058608A, so detailed description thereof will be omitted.

First, the imaging controller 21 acquires the imaging purpose input by the operator through the input device 15 (Step ST1), and specifies at least one radiation detector used for imaging (Step ST2). Here, since the imaging purpose is energy subtraction imaging, the first and second radiation detectors 5 and 6 included in the layer structure detector 4 are specified as radiation detectors to be used for imaging.

Next, the imaging controller 21 drives the radiation source 3 to perform radiography of the subject H under predetermined imaging conditions by operating the irradiation switch 19, and acquires a first radiation image G1 and a second radiation image G2 (Step ST3). Next, the subtraction unit 25 performs the energy subtraction processing using the first and second radiation images G1 and G2 (Step ST4). Accordingly, the bone part image Gb and the soft part image Gs of the subject H are derived.

The determination unit 23 specifies the subject region GH2 in the second radiation image G2 acquired by the second radiation detector 6 (Step ST5), and determines whether or not the dose is insufficient in the at least some pixels of the subject region GH2 (Step ST6). In a case in which the determination result in Step ST6 is "No", the determination unit 23 determines whether or not the dose is excessive due to at least a part of the region GH1 being saturated in the first radiation image G1 acquired by the first radiation detector 5 (Step ST7).

In a case in which the determination results in "Yes" in Step ST6 and Step ST7, the dose setting unit 24 derives a dose setting value for setting the pixel value of the subject region to a target value (Step ST8).

Then, the display controller 26 displays the bone part image Gb and the soft part image Gs and notifies of the determination result of the dose excess or insufficiency (determination result notification: Step ST9), and the processing ends. In addition, in a case in which the determination result in Step ST7 is "No", the display controller 26 displays the bone part image Gb and the soft part image Gs and notifies of the information representing that the dose is appropriate (Step ST10), and the processing ends.

As described above, in the present embodiment, the excess or insufficiency of the radiation in the radiography is determined based on at least one radiation image of the first radiation image G1 or the second radiation image G2 acquired by the first and second radiation detectors 5 and 6 included in the layer structure detector 4, and the determination result is output. Therefore, by performing the radiography again according to the result of the excess or insufficiency of the dose, it is possible to acquire a high-quality radiation image in a case in which the radiography is performed using the layer structure detector 4.

Here, since the second radiation detector 6 is located on a side far from the radiation source 3 in the layer structure detector 4, the amount of radiation irradiated onto the second radiation detector 6 is smaller than that of the first radiation detector 5. Therefore, in a case in which the imaging part is the chest, the dose may be insufficient in the mediastinum and the subdiaphragmatic region even though the dose in the lung field is sufficient. In the present embodiment, the second radiation image G2 acquired by the second radiation detector 6 is used as a radiation image for determination that is used for determining the excess or insufficiency of the radiation, and the excess or insufficiency of the dose is determined. Therefore, it is possible to accurately determine the dose insufficiency of the second radiation detector 6.

Further, the first radiation detector 5 on the side close to the radiation source 3 has a larger dose than the second radiation detector 6. Therefore, even in a case in which the second radiation image G2 is not insufficient in the dose, the pixel value on the high density side may be saturated in the first radiation image G1 acquired by the first radiation detector 5. In the present embodiment, in a case in which the second radiation image G2 is not insufficient in the dose, since the excess or insufficiency of the dose is determined using the first radiation image G1, it is possible to accurately determine the excess of the dose of the first radiation detector 5.

In addition, by notifying of the determination result of the dose excess or insufficiency, it is possible to easily recognize whether the dose is insufficient, excessive, or appropriate at the time of the radiography.

In addition, in a case in which a set value of the dose is derived and notified in a case in which there is excess or insufficiency of the dose, and the imaging is performed by using the dose set value notified at the time of the next imaging, it is possible to acquire a high-quality radiation image in which there is no excess or insufficiency in the dose, that is, in which the granular noise is small and the saturation of the pixel value is not present.

In the above-described embodiment, the excess or insufficiency of the dose is determined using both the first radiation image G1 and the second radiation image G2, but the present disclosure is not limited thereto. It may be determined only whether or not the dose is excessive by using only the first radiation image G1. In addition, it may be determined whether or not the dose is insufficient by using only the second radiation image G2.

In addition, the radiation in the embodiment described above is not particularly limited, and $\alpha$-rays or $\gamma$-rays can be used in addition to X-rays.

In addition, in the above-described embodiment, for example, the following various processors can be used as the hardware structure of processing units that execute various types of processing, such as the imaging controller 21, the device controller 22, the determination unit 23, the dose setting unit 24, the subtraction unit 25, and the display controller 26. As described above, the various processors include, in addition to the CPU that is a general-purpose processor which executes software (program) to function as various processing units, a programmable logic device (PLD) that is a processor of which a circuit configuration can be changed after manufacture, such as a field programmable gate array (FPGA), and a dedicated electrical circuit that is a processor having a circuit configuration which is designed for exclusive use to execute a specific processing, such as an application specific integrated circuit (ASIC).

One processing unit may be configured by one of these various processors, or may be configured by combining two or more processors of the same type or different types (for example, by combining a plurality of FPGAs or combining the CPU and the FPGA). Further, a plurality of processing units may be composed of one processor.

As an example of configuring the plurality of processing units by one processor, first, as represented by a computer of a client, a server, and the like, there is an aspect in which one processor is configured by a combination of one or more CPUs and software and this processor functions as a plurality of processing units. Second, as represented by a system on chip (SoC) or the like, there is an aspect of using a processor that realizes the function of the entire system including the plurality of processing units by one integrated circuit (IC) chip. In this way, various processing units are formed using one or more of the above-mentioned various processors as hardware structures.

Further, as the hardware structures of these various processors, more specifically, it is possible to use an electrical circuit (circuitry) in which circuit elements, such as semiconductor elements, are combined.

Hereinafter, description regarding supplementary notes of the present disclosure will be made.

(Supplementary Note 1)

A radiography control device comprising: at least one processor, in which the processor is configured to: acquire a plurality of radiation images from a plurality of radiation detectors by performing radiography in which a layer structure detector configured by stacking the plurality of radiation detectors is irradiated with radiation emitted from a radiation source and transmitted through an imaging target; determine excess or insufficiency of the radiation at a time of the radiography by using at least one radiation image of the plurality of radiation images as a radiation image for determination; and notify of a determination result of the excess or insufficiency.

(Supplementary Note 2)

The radiography control device according to Supplementary Note 1, in which the processor is configured to determine the excess or insufficiency of the radiation by using a radiation image acquired by one radiation detector other than a radiation detector closest to the radiation source among the plurality of radiation detectors as the radiation image for determination.

(Supplementary Note 3)

The radiography control device according to Supplementary Note 2, in which the processor is configured to: specify a subject region of the radiation image for determination; and determine that a dose of the radiation with which the one radiation detector is irradiated is insufficient in a case in which a pixel value of at least a part of the subject region is less than a reference pixel value or a granular noise amount of at least a part of the subject region exceeds a reference amount.

(Supplementary Note 4)

The radiography control device according to Supplementary Note 3, in which the processor is configured to: detect an artificial object region included in the radiation image for determination; and specify a region excluding the artificial object region as the subject region.

(Supplementary Note 5)

The radiography control device according to Supplementary Note 3 or 4, in which the processor is configured to: derive a minified image of the radiation image for determination; and determine whether or not the pixel value of at least a part of the subject region is less than the reference pixel value based on the minified image.

(Supplementary Note 6)

The radiography control device according to Supplementary Note 3 or 4, in which the processor is configured to determine whether or not the granular noise amount of at least a part of the subject region exceeds the reference amount, based on a high-frequency component of the radiation image for determination.

(Supplementary Note 7)

The radiography control device according to any one of Supplementary Notes 3 to 6, in which the processor is configured to, in a case in which it is determined that the dose of the radiation with which the one radiation detector is irradiated is not insufficient, determine the excess or insufficiency of the radiation by using a radiation image acquired by the radiation detector closest to the radiation source among the plurality of radiation detectors as an additional radiation image for determination.

(Supplementary Note 8)

The radiography control device according to Supplementary Note 7, in which the processor is configured to determine that the dose is excessive in a case in which a pixel value of at least a part of a region corresponding to the subject region in the additional radiation image for determination is saturated.

(Supplementary Note 9)

The radiography control device according to any one of Supplementary Notes 3 to 8, in which the processor is configured to: derive a dose setting value for setting the pixel value of the subject region to a target value in a case in which it is determined that the dose of the radiation is excessive or insufficient; and notify of the dose setting value.

(Supplementary Note 10)

A radiography control method comprising: acquiring a plurality of radiation images from a plurality of radiation detectors by performing radiography in which a layer structure detector configured by stacking the plurality of radiation detectors is irradiated with radiation emitted from a radiation source and transmitted through an imaging target; determining excess or insufficiency of the radiation at a time of the radiography by using at least one radiation image of the plurality of radiation images as a radiation image for determination; and notifying of a determination result of the excess or insufficiency.

(Supplementary Note 11)

A radiography control program causing a computer to execute: a procedure of acquiring a plurality of radiation images from a plurality of radiation detectors by performing radiography in which a layer structure detector configured by stacking the plurality of radiation detectors is irradiated with radiation emitted from a radiation source and transmitted through an imaging target; a procedure of determining excess or insufficiency of the radiation at a time of the radiography by using at least one radiation image of the plurality of radiation images as a radiation image for determination; and a procedure of notifying of a determination result of the excess or insufficiency.

Explanation of References

1: imaging apparatus
3: radiation source
4: layer structure detector
5, 6: radiation detector
7: radiation energy conversion filter
10: radiography control device
11: CPU
12: radiography control processing program
13: storage
14: display
15: input device
16: memory
17: network I/F
18: high voltage generator
19: irradiation switch
20: bus
21: radiation source controller
22: device controller
23: determination unit
24: dose setting unit
25: subtraction unit
26: display controller
40: histogram
41: direct radiation region
42: region of artificial object
45: thing
50: display screen
51: information representing that dose is appropriate at time of imaging

52: information representing that dose is insufficient at time of imaging
53, 55: information representing dose setting value
54: information representing that dose is excessive at time of imaging
P1, P2: profile
G1: first radiation image
G2: second radiation image
GH2: subject region
Gs: soft part image
Gb: bone part image

**Claims**

1. A radiography control device comprising:

   at least one processor,
   wherein the processor is configured to:

   acquire a plurality of radiation images from a plurality of radiation detectors by performing radiography in which a layer structure detector configured by stacking the plurality of radiation detectors is irradiated with radiation emitted from a radiation source and transmitted through an imaging target;
   determine excess or insufficiency of the radiation at a time of the radiography by using at least one radiation image of the plurality of radiation images as a radiation image for determination; and
   notify of a determination result of the excess or insufficiency.

2. The radiography control device according to claim 1,
   wherein the processor is configured to determine the excess or insufficiency of the radiation by using a radiation image acquired by one radiation detector other than a radiation detector closest to the radiation source among the plurality of radiation detectors as the radiation image for determination.

3. The radiography control device according to claim 2,
   wherein the processor is configured to:

   specify a subject region of the radiation image for determination; and
   determine that a dose of the radiation with which the one radiation detector is irradiated is insufficient in a case in which a pixel value of at least a part of the subject region is less than a reference pixel value or a granular noise amount of at least a part of the subject region exceeds a reference amount.

4. The radiography control device according to claim 3,
   wherein the processor is configured to:

   detect an artificial object region included in the radiation image for determination; and
   specify a region excluding the artificial object region as the subject region.

5. The radiography control device according to claim 3 or 4,
   wherein the processor is configured to:

   derive a minified image of the radiation image for determination; and
   determine whether or not the pixel value of at least a part of the subject region is less than the reference pixel value based on the minified image.

6. The radiography control device according to claim 3 or 4,
   wherein the processor is configured to determine whether or not the granular noise amount of at least a part of the subject region exceeds the reference amount, based on a high-frequency component of the radiation image for determination.

7. The radiography control device according to claim 3,
   wherein the processor is configured to, in a case in which it is determined that the dose of the radiation with which the one radiation detector is irradiated is not insufficient, determine the excess or insufficiency of the radiation by using a radiation image acquired by the radiation detector closest to the radiation source among the plurality of radiation detectors as an additional radiation image for determination.

8. The radiography control device according to claim 7,
wherein the processor is configured to determine that the dose is excessive in a case in which a pixel value of at least a part of a region corresponding to the subject region in the additional radiation image for determination is saturated.

9. The radiography control device according to claim 3,
wherein the processor is configured to:

derive a dose setting value for setting the pixel value of the subject region to a target value in a case in which it is determined that the dose of the radiation is excessive or insufficient; and
notify of the dose setting value.

10. A radiography control method comprising:

acquiring a plurality of radiation images from a plurality of radiation detectors by performing radiography in which a layer structure detector configured by stacking the plurality of radiation detectors is irradiated with radiation emitted from a radiation source and transmitted through an imaging target;
determining excess or insufficiency of the radiation at a time of the radiography by using at least one radiation image of the plurality of radiation images as a radiation image for determination; and
notifying of a determination result of the excess or insufficiency.

11. A radiography control program causing a computer to execute:

a procedure of acquiring a plurality of radiation images from a plurality of radiation detectors by performing radiography in which a layer structure detector configured by stacking the plurality of radiation detectors is irradiated with radiation emitted from a radiation source and transmitted through an imaging target;
a procedure of determining excess or insufficiency of the radiation at a time of the radiography by using at least one radiation image of the plurality of radiation images as a radiation image for determination; and
a procedure of notifying of a determination result of the excess or insufficiency.

# FIG. 1

RADIOGRAPHY
CONTROL DEVICE

# FIG. 2

## FIG. 3

RADIOGRAPHY CONTROL DEVICE — 10

IMAGING CONTROLLER — 21

DEVICE CONTROLLER — 22

DETERMINATION UNIT — 23

DOSE SETTING UNIT — 24

SUBTRACTION UNIT — 25

DISPLAY CONTROLLER — 26

## FIG. 4

FREQUENCY

40

Dmin    Dmax

PIXEL VALUE

## FIG. 5

# FIG. 6

# FIG. 7

## FIG. 8

## FIG. 9

# FIG. 10

Gs

Gb

50

DOSE IS APPROPRIATE. — 51

# FIG. 11

50

Gs

Gb

DOSE IS INSUFFICIENT. ←—52
DOSE SETTING VALUE IS ○. ←—53

# FIG. 12

50

Gs

Gb

DOSE IS EXCESSIVE. ←—54
DOSE SETTING VALUE IS △. ←—55

# FIG. 13

START

↓

ST1

AQCUIRE
IMAGING PURPOSE

↓

ST2

DETERMINE RADIATION
DETECTOR TO BE USED

↓

ST3

PERFORM RADIOGRAPHY

↓

ST4

ENERGY SUBTRACTION
PROCESSING

↓

ST5

SPECIFY SUBJECT REGION
IN SECOND RADIATION
IMAGE

↓

ST6

IS DOSE INSUFFICIENT? — YES

↓ NO

ST7

IS DOSE EXCESSIVE? — NO

↓ YES

ST8

DERIVE DOSE
SETTING VALUE

↓

ST9

NOTIFY OF
DETERMINATION RESULT

ST10

NOTIFY OF INFORMATION
REPRESENTING THAT DOSE
IS APPROPRIATE

↓

END

## INTERNATIONAL SEARCH REPORT

International application No.

**PCT/JP2023/042780**

### A. CLASSIFICATION OF SUBJECT MATTER

***A61B 6/00***(2024.01)i

FI: A61B6/00 520Z; A61B6/00 533; A61B6/00 550D; A61B6/00 550S

According to International Patent Classification (IPC) or to both national classification and IPC

### B. FIELDS SEARCHED

Minimum documentation searched (classification system followed by classification symbols)

A61B6/00-6/58; G01T1/00-1/40; G01T7/00-7/12; H05G1/26-1/54

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | JP 2018-023769 A (FUJIFILM CORPORATION) 15 February 2018 (2018-02-15) particularly, paragraphs [0008]-[0011], [0026]-[0066], [0102]-[0132], fig. 1-5, 13, 14 | 1-2, 10-11 |
| Y | | 3-9 |
| Y | JP 2015-043792 A (FUJIFILM CORPORATION) 12 March 2015 (2015-03-12) particularly, paragraphs [0036]-[0056], fig. 4 | 3-9 |
| Y | JP 2011-189082 A (CANON KABUSHIKI KAISHA) 29 September 2011 (2011-09-29) particularly, paragraphs [0044]-[0047], fig. 2, 3 | 3-9 |
| Y | JP 10-105701 A (FUJI PHOTO FILM CO., LTD.) 24 April 1998 (1998-04-24) particularly, paragraphs [0013], [0016], [0017], [0036] | 6 |
| Y | JP 2018-130336 A (CANON KABUSHIKI KAISHA) 23 August 2018 (2018-08-23) particularly, paragraphs [0018], [0021], [0030], fig. 2, 3 | 8 |
| A | JP 2020-193914 A (CANON KABUSHIKI KAISHA) 03 December 2020 (2020-12-03) entire text, all drawings | 1-11 |

☑ Further documents are listed in the continuation of Box C.  ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **07 February 2024** | **20 February 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (July 2022)

## INTERNATIONAL SEARCH REPORT

| International application No. |
| --- |
| **PCT/JP2023/042780** |

### C. DOCUMENTS CONSIDERED TO BE RELEVANT

| Category* | Citation of document, with indication, where appropriate, of the relevant passages | Relevant to claim No. |
| --- | --- | --- |
| A | JP 2019-058608 A (FUJIFILM CORPORATION) 18 April 2019 (2019-04-18)<br>entire text, all drawings | 1-11 |
| A | JP 2018-033745 A (FUJIFILM CORPORATION) 08 March 2018 (2018-03-08)<br>entire text, all drawings | 1-11 |

Form PCT/ISA/210 (second sheet) (July 2022)

## INTERNATIONAL SEARCH REPORT
### Information on patent family members

International application No.

**PCT/JP2023/042780**

| Patent document cited in search report | | | Publication date (day/month/year) | Patent family member(s) | | | Publication date (day/month/year) |
|---|---|---|---|---|---|---|---|
| JP | 2018-023769 | A | 15 February 2018 | US | 2018/0031714 | A1 | |
| | | | | particularly, paragraphs [0009]-[0012], [0051]-[0091], [0127]-[0157], fig. 1-5, 13, 14 | | | |
| JP | 2015-043792 | A | 12 March 2015 | US | 2016/0171691 | A1 | |
| | | | | particularly, paragraphs [0050]-[0070], fig. 4 | | | |
| | | | | WO | 2015/029399 | A1 | |
| JP | 2011-189082 | A | 29 September 2011 | US | 2011/0226956 | A1 | |
| | | | | particularly, paragraphs [0056]-[0059], fig. 2, 3 | | | |
| | | | | EP | 2366331 | A1 | |
| | | | | EP | 2649939 | A1 | |
| | | | | CN | 102188259 | A | |
| | | | | CN | 103284739 | A | |
| JP | 10-105701 | A | 24 April 1998 | (Family: none) | | | |
| JP | 2018-130336 | A | 23 August 2018 | US | 2018/0232872 | A1 | |
| | | | | particularly, paragraphs [0021], [0024], [0034], fig. 2, 3 | | | |
| JP | 2020-193914 | A | 03 December 2020 | US | 2022/0075085 | A1 | |
| | | | | entire text, all drawings | | | |
| | | | | WO | 2020/241062 | A1 | |
| JP | 2019-058608 | A | 18 April 2019 | US | 2019/0090837 | A1 | |
| | | | | entire text, all drawings | | | |
| JP | 2018-033745 | A | 08 March 2018 | US | 2018/0061023 | A1 | |
| | | | | entire text, all drawings | | | |

Form PCT/ISA/210 (patent family annex) (July 2022)

**EP 4 659 674 A1**

## REFERENCES CITED IN THE DESCRIPTION

**Patent documents cited in the description**

- JP 2019058608 A

- JP 2018033745 A